# NEW EUROPEAN PATENT SPECIFICATION

(11) **EP 3 131 928 B2**
(45) Date of publication and mention of the opposition decision: **05.04.2023**
(45) Mention of the grant of the patent: 20.11.2019
(21) Application number: 15719420.0
(22) Date of filing: 12.04.2015
(51) Int. Cl.: C07K 16/28, C07K 16/46

(54) **TRIFUNCTIONAL ANTIGEN-BINDING MOLECULE**
TRIFUNKTIONELLES ANTIGEN-BINDENDES MOLEKÜL
MOLÉCULE DE LIAISON À L'ANTIGÈNE TRIFONCTIONNELLE

(30) Priority: 13.04.2014 EP 14164523
(43) Date of publication of application: 22.02.2017
(73) Proprietor: Affimed GmbH, 69120 Heidelberg (DE)
(72) Inventor: LITTLE, Melvyn, 25826 St. Peter-Ording (DE); ZHUKOVSKY, Eugene, 68165 Mannheim (DE); ESER, Markus, 40593 Düsseldorf (DE); WEICHEL, Michael, 65474 Bischofsheim (DE); GANTKE, Thorsten, 68535 Edingen-Neckarhausen (DE); REUSCH, Uwe, 67487 Maikammer (DE); ELLWANGER, Kristina, 69120 Heidelberg (DE); LE GALL, Fabrice, 55122 Mainz (DE)
(74) Representative: Schüssler, Andrea
(86) International application number: PCT/EP2015/057919
(87) International publication number: WO 2015/158636

(56) References cited:
- WO-A1-2010/136172
- WO-A2-03/025018
- R. CASTOLDI ET AL: "Molecular characterization of novel trispecific ErbB-cMet-IGF1R antibodies and their antigen-binding properties", PROTEIN ENGINEERING DESIGN AND SELECTION, vol. 25, no. 10, 1 October 2012 (2012-10-01), pages 551-560, XP055041659, ISSN: 1741-0126, DOI: 10.1093/protein/gzs048
- KUEGLER MARKUS ET AL: "A recombinant trispecific single-chain Fv derivative directed against CD123 and CD33 mediates effective elimination of acute myeloid leukaemia cells by dual targeting", BRITISH JOURNAL OF HAEMATOLOGY, WILEY-BLACKWELL PUBLISHING LTD, GB, vol. 150, no. 5, 1 September 2010 (2010-09-01), pages 574-586, XP009142424, ISSN: 0007-1048, DOI: 10.1111/J.1365-2141.2010.08300.X [retrieved on 2010-07-16]
- CUESTA A M ET AL: "Multivalent antibodies: when design surpasses evolution", TRENDS IN BIOTECHNOLOGY, ELSEVIER PUBLICATIONS, CAMBRIDGE, GB, vol. 28, no. 7, 1 July 2010 (2010-07-01), pages 355-362, XP027102411, ISSN: 0167-7799 [retrieved on 2010-05-04]
- HUDSON P J ET AL: "High avidity scFv multimers; diabodies and triabodies", JOURNAL OF IMMUNOLOGICAL METHODS, ELSEVIER SCIENCE PUBLISHERS B.V.,AMSTERDAM, NL, vol. 231, no. 1-2, 10 December 1999 (1999-12-10), pages 177-189, XP004187644, ISSN: 0022-1759, DOI: 10.1016/S0022-1759(99)00157-X
- X.-B. WANG: "A New Recombinant Single Chain Trispecific Antibody Recruits T Lymphocytes to Kill CEA (Carcinoma Embryonic Antigen) Positive Tumor Cells In Vitro Efficiently", JOURNAL OF BIOCHEMISTRY, vol. 135, no. 4, 1 April 2004 (2004-04-01) , pages 555-565, XP055132180, ISSN: 0021-924X, DOI: 10.1093/jb/mvh065
- Ishiyama, T et al., "The increase of CD5L0W+NK cells in patients with multiple myeloma and plasmacytoma" Anticancer Research, Vol, 14, March 1994
- Le Gall, Fabrice et al., "Effect of linker sequences between the antibody variable domains on the formation, stability and biological activity of a bispecific tandem diabody" Protein Engineering, Design & Selection, Vol. 17, 04 May 2004
- Kontermann, Roland et al., "Bispecific antibodies" Drug Discover Today. Vol. 20, 26 February 2015
- Kugler, Markus et al., "A recombinant trispecific single-chain Fv derivative directed against CD123 and CD33 mediates effective elimination of acute myeloid leukaemia cells by dual targeting" Britisch Journal of Haemotology, Vol. 150, 16 July 2010
- Kato et al, Annals of Clinical Pathology, 2016, 4(5): 1082
- Novus Biologicals website article dated 5 January 2015 -https.//www. novusbio.com/antibody-news/antibodies/cd16 -find-me-on-macrophages-neutrophils-and-nk -cells

### SEQUENCE LISTING

<110> Affimed
<120> Trifunctional Antigen Binding Molecule
<130> A 3264PCT
<150> EP14164523.4
   <151> 2014-04-13
<160> 6
<170> Patent In version 3.5
<210> 1
   <211> 509
   <212> PRT
   <213> artificial sequence
<220>
   <223> polypeptide chain
<400> 1
   Gln Val Gln Leu Gln Gln Ser Gly Ala Glu Leu Ala Arg Pro Gly Ala
   1               5                   10                  15
   Ser Val Lys Met Ser Cys Lys Ala Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser Gly Gly Ser Gly Gly Ser Gln Val Gln
           115                 120                 125
   Leu Gln Gln Ser Gly Ala Glu Leu Ala Arg Pro Gly Ala Ser Val Lys
       130                 135                 140
   Met Ser Cys Lys Ala Ser Gly Tyr Thr Phe Thr Arg Tyr Thr Met His
   145                 150                 155                 160
   Trp Val Lys Gln Arg Pro Gly Gln Gly Leu Glu Trp Ile Gly Tyr Ile
   165 170 175
   Asn Pro Ser Arg Gly Tyr Thr Asn Tyr Asn Gln Lys Phe Lys Asp Lys
   180 185 190
   Ala Thr Leu Thr Thr Asp Lys Ser Ser Ser Thr Ala Tyr Met Gln Leu
   195 200 205
   Ser Ser Leu Thr Ser Glu Asp Ser Ala Val Tyr Tyr Cys Ala Arg Tyr
   210 215 220
   Tyr Asp Asp His Tyr Cys Leu Asp Tyr Trp Gly Gln Gly Thr Thr Leu
   225 230 235 240
   Thr Val Ser Ser Gly Gly Ser Gly Gly Ser Gly Gly Ser Gln Val Gln
   245 250 255
   Leu Gln Gln Ser Gly Ala Glu Leu Ala Arg Pro Gly Ala Ser Val Lys
   260 265 270
   Met Ser Cys Lys Ala Ser Gly Tyr Thr Phe Thr Thr Tyr Thr Ile His
   275 280 285
   Trp Val Arg Gln Arg Pro Gly His Asp Leu Glu Trp Ile Gly Tyr Ile
   290 295 300
   Asn Pro Ser Ser Gly Tyr Ser Asp Tyr Asn Gln Asn Phe Lys Gly Lys
   305 310 315 320
   Thr Thr Leu Thr Ala Asp Lys Ser Ser Asn Thr Ala Tyr Met Gln Leu
   325 330 335
   Asn Ser Leu Thr Ser Glu Asp Ser Ala Val Tyr Tyr Cys Ala Arg Arg
   340 345 350
   Ala Asp Tyr Gly Asn Tyr Glu Tyr Thr Trp Phe Ala Tyr Trp Gly Gln
   355 360 365
   Gly Thr Thr Val Thr Val Ser Ser Gly Gly Ser Gly Gly Ser Gly Gly
   370 375 380
   Ser Gly Gly Ser Gly Gly Ser Asp Ile Val Met Thr Gln Ser Pro Lys
   385 390 395 400
   Phe Met Ser Thr Ser Val Gly Asp Arg Val Thr Val Thr Cys Lys Ala
   405 410 415
   Ser Gln Asn Val Gly Thr Asn Val Ala Trp Phe Gln Gln Lys Pro Gly
   420 425 430
   Gln Ser Pro Lys Val Leu Ile Tyr Ser Ala Ser Tyr Arg Tyr Ser Gly
   435 440 445
   Val Pro Asp Arg Phe Thr Gly Ser Gly Ser Gly Thr Asp Phe Thr Leu
   450 455 460
   Thr Ile Ser Asn Val Gln Ser Glu Asp Leu Ala Glu Tyr Phe Cys Gln
   465 470 475 480
   Gln Tyr His Thr Tyr Pro Leu Thr Phe Gly Gly Gly Thr Lys Leu Glu
   485 490 495
   Ile Asn Ala Ala Ala Gly Ser His His His His His His
   500 505
<210> 2
   <211> 490
   <212> PRT
   <213> artificial sequence
<220>
   <223> polypeptide chain
<400> 2
   Gln Ile Val Leu Thr Gln Ser Pro Ala Ile Met Ser Ala Ser Pro Gly
   1               5                   10                  15
   Glu Lys Val Thr Met Thr Cys Ser Ala Ser Ser Ser Val Ser Tyr Met
               20                  25                  30
   Asn Trp Tyr Gln Gln Lys Ser Gly Thr Ser Pro Lys Arg Trp Ile Tyr
           35                  40                  45
   Asp Thr Ser Lys Leu Ala Ser Gly Val Pro Ala His Phe Arg Gly Ser
       50                  55                  60
   Gly Ser Gly Thr Ser Tyr Ser Leu Thr Ile Ser Gly Met Glu Ala Glu
   65                  70                  75                  80
   Asp Ala Ala Thr Tyr Tyr Cys Gln Gln Trp Ser Ser Asn Pro Phe Thr
                   85                  90                  95
   Phe Gly Ser Gly Thr Lys Leu Glu Ile Asn Gly Gly Ser Gly Gly Ser
               100                 105                 110
   Gln Ile Val Leu Thr Gln Ser Pro Ala Ile Met Ser Ala Ser Pro Gly
               115                 120                 125
   Glu Lys Val Thr Met Thr Cys Ser Ala Ser Ser Ser Val Ser Tyr Met
       130                 135                 140
   Asn Trp Tyr Gln Gln Lys Ser Gly Thr Ser Pro Lys Arg Trp Ile Tyr
   145                 150                 155                 160
   Asp Thr Ser Lys Leu Ala Ser Gly Val Pro Ala His Phe Arg Gly Ser
                   165                 170                 175
   Gly Ser Gly Thr Ser Tyr Ser Leu Thr Ile Ser Gly Met Glu Ala Glu
               180                 185                 190
   Asp Ala Ala Thr Tyr Tyr Cys Gln Gln Trp Ser Ser Asn Pro Phe Thr
           195                 200                 205
   Phe Gly Ser Gly Thr Lys Leu Glu Ile Asn Gly Gly Ser Gly Gly Ser
       210                 215                 220
   Gly Gly Ser Gln Val Gln Leu Gln Gln Ser Gly Ala Glu Leu Val Arg
   225                 230                 235                 240
   Pro Gly Ser Ser Val Lys Ile Ser Cys Lys Ala Ser Gly Tyr Ala Phe
                   245                 250                 255
   Ser Ser Tyr Trp Met Asn Trp Val Lys Gln Arg Pro Gly Gln Gly Leu
               260                 265                 270
   Glu Trp Ile Gly Gln Ile Trp Pro Gly Asp Gly Asp Thr Asn Tyr Asn
           275                 280                 285
   Gly Lys Phe Lys Gly Lys Ala Thr Leu Thr Ala Asp Glu Ser Ser Ser
       290                 295                 300
   Thr Ala Tyr Met Gln Leu Ser Ser Leu Ala Ser Glu Asp Ser Ala Val
   305                 310                 315                 320
   Tyr Phe Cys Ala Arg Arg Glu Thr Thr Thr Val Gly Arg Tyr Tyr Tyr
                   325                 330                 335
   Ala Met Asp Tyr Trp Gly Gln Gly Thr Ser Val Thr Val Ser Ser Gly
               340                 345                 350
   Gly Ser Gly Gly Ser Gly Gly Ser Gly Gly Ser Gly Gly Ser Asp Ile
                   355                 360                 365
   Leu Leu Thr Gln Thr Pro Ala Ser Leu Ala Val Ser Leu Gly Gln Arg
       370                 375                 380
   Ala Thr Ile Ser Cys Lys Ala Ser Gln Ser Val Asp Tyr Asp Gly Asp
   385                 390                 395                 400
   Ser Tyr Leu Asn Trp Tyr Gln Gln Ile Pro Gly Gln Pro Pro Lys Leu
                   405                 410                 415
   Leu Ile Tyr Asp Ala Ser Asn Leu Val Ser Gly Ile Pro Pro Arg Phe
               420                 425                 430
   Ser Gly Ser Gly Ser Gly Thr Asp Phe Thr Leu Asn Ile His Pro Val
           435                 440                 445
   Glu Lys Val Asp Ala Ala Thr Tyr His Cys Gln Gln Ser Thr Glu Asp
       450                 455                 460
   Pro Trp Thr Phe Gly Gly Gly Thr Lys Leu Glu Ile Asn Ala Ala Ala
   465                 470                 475                 480
   Gly Ser Asp Tyr Lys Asp Asp Asp Asp Lys
                   485                 490
<210> 3
   <211> 513
   <212> PRT
   <213> artificial sequence
<220>
   <223> polypeptide chain
<400> 3
   Gln Val Gln Leu Gln Gln Ser Gly Ala Glu Leu Ala Arg Pro Gly Ala
   1               5                   10                  15
   Ser Val Lys Met Ser Cys Lys Ala Ser Gly Tyr Thr Phe Thr Thr Tyr
               20                  25                  30
   Thr Ile His Trp Val Arg Gln Arg Pro Gly His Asp Leu Glu Trp Ile
           35                  40                  45
   Gly Tyr Ile Asn Pro Ser Ser Gly Tyr Ser Asp Tyr Asn Gln Asn Phe
       50                  55                  60
   Lys Gly Lys Thr Thr Leu Thr Ala Asp Lys Ser Ser Asn Thr Ala Tyr
   65                  70                  75                  80
   Met Gln Leu Asn Ser Leu Thr Ser Glu Asp Ser Ala Val Tyr Tyr Cys
                   85                  90                  95
   Ala Arg Arg Ala Asp Tyr Gly Asn Tyr Glu Tyr Thr Trp Phe Ala Tyr
               100                 105                 110
   Trp Gly Gln Gly Thr Thr Val Thr Val Ser Ser Gly Gly Ser Gly Gly
           115                 120                 125
   Ser Gln Val Gln Leu Gln Gln Ser Gly Ala Glu Leu Val Arg Pro Gly
       130                 135                 140
   Ser Ser Val Lys Ile Ser Cys Lys Ala Ser Gly Tyr Ala Phe Ser Ser
   145                 150                 155                 160
   Tyr Trp Met Asn Trp Val Lys Gln Arg Pro Gly Gln Gly Leu Glu Trp
                   165                 170                 175
   Ile Gly Gln Ile Trp Pro Gly Asp Gly Asp Thr Asn Tyr Asn Gly Lys
               180                 185                 190
   Phe Lys Gly Lys Ala Thr Leu Thr Ala Asp Glu Ser Ser Ser Thr Ala
           195                 200                 205
   Tyr Met Gln Leu Ser Ser Leu Ala Ser Glu Asp Ser Ala Val Tyr Phe
       210                 215                 220
   Cys Ala Arg Arg Glu Thr Thr Thr Val Gly Arg Tyr Tyr Tyr Ala Met
   225                 230                 235                 240
   Asp Tyr Trp Gly Gln Gly Thr Ser Val Thr Val Ser Ser Gly Gly Ser
                   245                 250                 255
   Gly Gly Ser Gly Gly Ser Gln Val Gln Leu Gln Gln Ser Gly Ala Glu
               260                 265                 270
   Leu Ala Arg Pro Gly Ala Ser Val Lys Met Ser Cys Lys Ala Ser Gly
           275                 280                 285
   Tyr Thr Phe Thr Arg Tyr Thr Met His Trp Val Lys Gln Arg Pro Gly
       290                 295                 300
   Gln Gly Leu Glu Trp Ile Gly Tyr Ile Asn Pro Ser Arg Gly Tyr Thr
   305                 310                 315                 320
   Asn Tyr Asn Gln Lys Phe Lys Asp Lys Ala Thr Leu Thr Thr Asp Lys
   325 330 335
   Ser Ser Ser Thr Ala Tyr Met Gln Leu Ser Ser Leu Thr Ser Glu Asp
   340 345 350
   Ser Ala Val Tyr Tyr Cys Ala Arg Tyr Tyr Asp Asp His Tyr Cys Leu
   355 360 365
   Asp Tyr Trp Gly Gln Gly Thr Thr Leu Thr Val Ser Ser Gly Gly Ser
   370 375 380
   Gly Gly Ser Gly Gly Ser Gly Gly Ser Gly Gly Ser Gln Ile Val Leu
   385 390 395 400
   Thr Gln Ser Pro Ala Ile Met Ser Ala Ser Pro Gly Glu Lys Val Thr
   405 410 415
   Met Thr Cys Ser Ala Ser Ser Ser Val Ser Tyr Met Asn Trp Tyr Gln
   420 425 430
   Gln Lys Ser Gly Thr Ser Pro Lys Arg Trp Ile Tyr Asp Thr Ser Lys
   435 440 445
   Leu Ala Ser Gly Val Pro Ala His Phe Arg Gly Ser Gly Ser Gly Thr
   450 455 460
   Ser Tyr Ser Leu Thr Ile Ser Gly Met Glu Ala Glu Asp Ala Ala Thr
   465 470 475 480
   Tyr Tyr Cys Gln Gln Trp Ser Ser Asn Pro Phe Thr Phe Gly Ser Gly
   485 490 495
   Thr Lys Leu Glu Ile Asn Ala Ala Ala Gly Ser His His His His His
   500 505 510
   His
<210> 4
   <211> 486
   <212> PRT
   <213> artificial sequence
<220>
   <223> polypeptide chain
<400> 4
   Asp Ile Leu Leu Thr Gln Thr Pro Ala Ser Leu Ala Val Ser Leu Gly
   1 5 10 15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Asn Gly
               100                 105                 110
   Gly Ser Gly Gly Ser Asp Ile Val Met Thr Gln Ser Pro Lys Phe Met
           115                 120                 125
   Ser Thr Ser Val Gly Asp Arg Val Thr Val Thr Cys Lys Ala Ser Gln
       130                 135                 140
   Asn Val Gly Thr Asn Val Ala Trp Phe Gln Gln Lys Pro Gly Gln Ser
   145                 150                 155                 160
   Pro Lys Val Leu Ile Tyr Ser Ala Ser Tyr Arg Tyr Ser Gly Val Pro
                   165                 170                 175
   Asp Arg Phe Thr Gly Ser Gly Ser Gly Thr Asp Phe Thr Leu Thr Ile
               180                 185                 190
   Ser Asn Val Gln Ser Glu Asp Leu Ala Glu Tyr Phe Cys Gln Gln Tyr
           195                 200                 205
   His Thr Tyr Pro Leu Thr Phe Gly Gly Gly Thr Lys Leu Glu Ile Asn
       210                 215                 220
   Gly Gly Ser Gly Gly Ser Gly Gly Ser Gln Val Gln Leu Gln Gln Ser
   225                 230                 235                 240
   Gly Ala Glu Leu Ala Arg Pro Gly Ala Ser Val Lys Met Ser Cys Lys
                   245                 250                 255
   Ala Ser Gly Tyr Thr Phe Thr Arg Tyr Thr Met His Trp Val Lys Gln
                   260                 265                 270
   Arg Pro Gly Gln Gly Leu Glu Trp Ile Gly Tyr Ile Asn Pro Ser Arg
               275                 280                 285
   Gly Tyr Thr Asn Tyr Asn Gln Lys Phe Lys Asp Lys Ala Thr Leu Thr
       290                 295                 300
   Thr Asp Lys Ser Ser Ser Thr Ala Tyr Met Gln Leu Ser Ser Leu Thr
   305                 310                 315                 320
   Ser Glu Asp Ser Ala Val Tyr Tyr Cys Ala Arg Tyr Tyr Asp Asp His
                   325                 330                 335
   Tyr Cys Leu Asp Tyr Trp Gly Gln Gly Thr Thr Leu Thr Val Ser Ser
               340                 345                 350
   Gly Gly Ser Gly Gly Ser Gly Gly Ser Gly Gly Ser Gly Gly Ser Gln
           355                 360                 365
   Ile Val Leu Thr Gln Ser Pro Ala Ile Met Ser Ala Ser Pro Gly Glu
       370                 375                 380
   Lys Val Thr Met Thr Cys Ser Ala Ser Ser Ser Val Ser Tyr Met Asn
   385                 390                 395                 400
   Trp Tyr Gln Gln Lys Ser Gly Thr Ser Pro Lys Arg Trp Ile Tyr Asp
                   405                 410                 415
   Thr Ser Lys Leu Ala Ser Gly Val Pro Ala His Phe Arg Gly Ser Gly
               420                 425                 430
   Ser Gly Thr Ser Tyr Ser Leu Thr Ile Ser Gly Met Glu Ala Glu Asp
           435                 440                 445
   Ala Ala Thr Tyr Tyr Cys Gln Gln Trp Ser Ser Asn Pro Phe Thr Phe
       450                 455                 460
   Gly Ser Gly Thr Lys Leu Glu Ile Asn Ala Ala Ala Gly Ser Trp Ser
   465                 470                 475                 480
   His Pro Gln Phe Glu Lys
                   485
<210> 5
   <211> 8
   <212> PRT
   <213> artificial sequence
<220>
   <223> Tag-sequence
<400> 5
   Asp Tyr Lys Asp Asp Asp Asp Lys
   1               5
<210> 6
   <211> 8
   <212> PRT
   <213> artificial sequence
<220>
   <223> Tag-sequence
<400> 6
   Trp Ser His Pro Gln Phe Glu Lys
   1               5

## Description

The present invention relates to a multifunctional, for example trifunctional, antigen-binding molecule and its therapeutic application, for example in immunotherapy. The molecule is a Fv-antibody derivative. The invention relates to dimeric antigen binding molecules.

Bispecific, i.e. bifunctional, antibodies can be used to engage two different therapeutic targets or perform two distinct functions. Such antibodies can be used for example to recruit an immune effector cell, e.g. T- or NK-cell, towards a particular target cell. Various antibody-fragment based molecules are known and under investigation, for example for cancer therapy.

Bifunctional and dimeric antibodies can be constructed using only antibody variable domains. For example, the linker sequence between the VH and VL domains can be shortened to such an extent that they cannot fold over and bind one another in an intramolecular fashion. Such short linkers, e.g. 2-12 residues, prevent said folding of a scFv molecule and favor intermolecular VH-VL pairings between complementary variable domains of different polypeptide chains forming a dimeric "diabody" (Holliger et al., 1993, Proc. Natl. Acad. Sci. USA 90, 6444-6448). Such diabody can be used for bifunctional antibodies, which are obtained by non-covalent association of two single-chain polypeptide fusion products, each consisting of the VH domain from one antibody connected by a short linker to the VL domain of another antibody.

WO 03/025018 discloses a bispecific and multimeric antigen-binding molecule which structure is formed by identical single-chain polypeptides with at least four binding domains. A VH and a VL domain at a terminal part of each polypeptide chain are linked by a short linker and associate intermolecularly with the corresponding VH and VL domains of another polypeptide chain, while the other VH and VL domains of each polypeptide chain bind intramolecularly to one another within the same chain resulting in an antigen-binding scFv unit. Such constructs are homodimers, i.e. they consist of identical single-chain polypeptides associated with one another.

Different IgG-like TriMAbs (trispecific, trivalent and tetravalent antibodies) directed against cell surface tumor antigens have been described (R. Castoldi et al., Protein Engineering, Design & Selection, 25(10):551-559, 2012; WO 2010/136172).

A single-chain trispecific and trivalent Fv-antibody has been disclosed (X-B Wang et al., J. Biochem, 135, 555-565, 2004).

Provided herein are multifunctional antigen-binding molecules, which are at least trifunctional. In some embodiments the trifunctional antigen-binding molecule is at least trispecific, i.e. has specificity for at least three different antigen epitopes.

The antigen-binding molecule according to the invention is a Fv-derivative which comprises only variable (Fv) antibody domains, but is devoid of constant antibody domains. The variable (Fv) antibody domains of the antigen-binding molecule are linked with one another by a peptide linker or a peptide bond.

In some embodiments the trispecific antigen-binding moelcule is at least tetravalent. "Tetravalent" means that the antigen-binding molecule comprises four antigen-binding sites, wherein each of the antigen-binding sites comprises a VH/VL pair having a variable heavy chain (VH) domain and a variable light chain (VL) domain of the same antigen epitope specificity associated with one another. Thus, such tetravalent antigen-binding molecule comprises at least eight variable antibody domains, namely four variable heavy chain (VH) domains and four variable light chain (VL) domains. The trispecific and tetravalent antigen-binding molecule comprises an antigen-binding site having specificity against a first antigen epitope, an antigen-binding site having specificity against a second antigen epitope and two antigen-binding sites having specificity against a third antigen epitope. Thus, this trispecific and tetravalent antigen-binding molecule has different specificities for three different antigen epitopes. For example, such antigen-binding molecule comprises a first antigen-binding site having specificity against a first antigen epitope, a second antigen-binding site having specificity against a second antigen epitope, a third and a fourth antigen-binding sites having specificity against a third antigen epitope. The antigen-binding molecule is heterodimeric, i.e. comprises two different polypeptide chains, wherein these two polypeptide chains differ in at least one variable domain, e.g. one polypeptide chain comprises only a VH domain and the other one comprises only the respective VL domain of the same antigen epitope specificity.

Because the tetravalent antigen-binding molecule comprises eight antibody variable domains its molecular weight is above 100 kDa which results in a longer half-life of such a molecule compared with trivalent and trispecific single-chain Fv molecules.

Further, each trispecific and tetravalent antigen-binding molecule comprises two antigen-binding sites having specificity for the same antigen epitope. Thereby the avidity is increased, i.e. the strength of interaction between the antigen epitope and antigen-binding molecule. Advantages of the higher avidity are increased stability of interaction and retention on the target. For example, if the target is a cytotoxic immune effector cell such as a T-cell or a NK-cell, the higher avidity can result in an increased cytolytic potential of the antigen-binding molecule. In another example, if the target is a tumor cell, the higher avidity improves the retention time on the target and reduces the off-rates from the target. In a certain embodiment of the invention, the trispecific and tetravalent antigen-binding molecule comprises a first and a second antigen-binding sites specific for two different antigen epitopes of the same kind of tumor cell and a third and a fourth antigen binding sites specific for an antigen epitope on an immune effector cell, such as T-cell or NK-cell. Such an antigen-binding molecule leads to an increased specificity as well as avidity for a particular kind of tumor cell and to an increased avidity for activating a receptor on the immune effector celll which results in an advantageously increased specific cytolytic potential of the antigen-binding molecule. The binding to two distinct tumor antigen epitopes leads to an increase in targeting specificity and to an extension of the therapeutic window by reducing off-target toxicities. Importantly, despite the structural complexity, such trispecific and tetravalent antigen-binding molecule according to the invention is stable.

Therefore, the antigen-binding molecule according to the invention can be utilized in different ways for redirecting the cytotoxic potential of immune effector cells to destroy tumor cells or infectious agents. In some embodiments the trispecific antigen-binding molecule may bind to two different antigen epitopes on a target. For example, the two different epitopes may be on the same antigen to prevent escape mutants or to enhance efficacy or the two epitopes may be on two different antigens of the target. In other embodiments the trispecific antigen-binding molecule may bind to two different antigen epitopes on immune effector cells. For example, a first antigen-binding site has specificity for an activating receptor, e.g. CD16A or CD3, and a second antigen-binding site has specificity for a co-stimulatory receptor, e.g,. CD137 or CD28. In another example, a first antigen-binding site has specificity for CD16A and a second antigen-binding site for another activating receptor on NK cells, e.g. NKG2D, DNAM, NCRs).

In another embodiment the trispecific antigen-binding molecule has a first antigen-binding site having specificity for an antigen epitope on a tumor cell, a second antigen-binding site having specificity for an antigen epitope on an immune effector cell and a third antigen-binding site having specificity for an antigen epitope on a soluble protein selected from the group of growth factors, cytokines, chemokines, mitogens and albumins. Examples of such a soluble protein are IL-6, BAFF, APRIL, TGF-beta, IL-10, VEGF-A, HB-EGF, angiopoetin-2 and human serum albumin (HSA).

In an alternative embodiment the antigen-binding molecule has one antigen-binding site having specificity for an antigen epitope of an antigen present on one type of cell and three antigen-binding sites having specificities of antigen epitopes on one or more other types of cells.

"Effector cells" are cells of the immune system which can stimulate or trigger cytotoxicity, phagocytosis, antigen presentation, cytokine release. Such effector cells are, for example but not limited to, T cells, natural killer (NK) cells, granulocytes, monocytes, macrophages, dendritic cells, and antigen-presenting cells. Examples of suitable specificities for effector cells include but are not limited to CD2, CD3 and CD3 subunits such as CD3ε, CD5, CD28 and other components of the T-cell receptor (TCR) for T cells; CD16 CD16A, CD25, CD38, CD44, CD56, CD69, CD94, CD335 (NKp46), CD336 (NKp44), CD337 (NKp30), NKp80, NKG2C and NKG2D, DNAM, NCRs for NK cells; CD18, CD64 and CD89 for granulocytes; CD18, CD32, CD64, CD89 and mannose receptor for monocytes and macrophages; CD64 and mannose receptor for dendritic cells; as well as CD35. In certain embodiments of the invention those specificities, i.e. cell surface molecules, of effector cells are suitable for mediating cell killing upon binding of a trispecific antigen-binding molecule to such cell surface molecule and, thereby, inducing cytolysis or apoptosis.

CD3 antigen is associated with the T-cell receptor complex on T-cells. In the case where specificity for an effector cell is CD3, the binding of the antigen-binding molecule according to the invention to CD3 triggers the cytotoxic activity of T-cells. By binding of the antigen-binding molecule to CD3 and to a target cell, e.g. tumor cell, cell lysis of the target cell may be induced.

The CD16A (FcyIIIA) antigen is a receptor expressed on the surface of NK cells. NK cells possess an inherent cytoloytic activity and by binding of the antigen-binding molecule according to the invention to CD16 or CD16A the cytotoxic activity of NK cell towards the target can be triggered.

"Target" is the site on which the antigen epitope is located and to which the antigen-binding molecule should bind to. Examples of targets are cells, infectious agents such as viral or bacterial pathogens, for example dengue virus, herpes simplex, influenza virus, HIV, HCV or cells carrying autoimmune targets such as IL-2/IL2R, an autoimmune marker or an autoimmune antigen or tumor cells. In embodiments, wherein at least one of the antigen-binding sites has specificity for an effector cell, the target can be a tumor cell to which the effector cell should be redirected to induce or trigger the respective biological, e.g. immune, response.

Suitable specificities for tumor cells may be tumor antigens and cell surface antigens on the respective tumor cell, for example specific tumor markers. The term "tumor antigen" as used herein comprises tumor associated antigen (TAA) and tumor specific antigen (TSA). A "tumor associated antigen" (TAA) as used herein refers to a protein which is present on tumor cells, and on normal cells during fetal life (once-fetal antigens), and after birth in selected organs, but at much lower concentration than on tumor cells. A TAA may also be present in the stroma in the vicinity of the tumor cell but expressed at lower amounts in the stroma elsewhere in the body. In contrast, the term "tumor specific antigen" (TSA) refers to a protein expressed by tumor cells. The term "cell surface antigen" refers to a molecule any antigen or fragment thereof capable of being recognized by an antibody on the surface of a cell. Examples of specificities for tumor cells include but are not limited to CD19, CD20, CD26, CD29, CD30, CD33, CD52, CD200, CD267, EGFR, EGFR2, EGFR3, EGFRvIII, HER2, HER3, IGFR, IGF-1R, Ep-CAM, PLAP, Thomsen-Friedenreich (TF) antigen, TNFRSF17, gpA33, MUC-1 (mucin), IGFR, CD5, IL4-R alpha, IL13-R, FcεRI, MHCl/peptide complexes and IgE.

Antigen-binding molecules according to the invention, wherein the tumor specificity is towards CD19 antigen may be used for immunotherapy of B-cell malignancies, because the CD19 antigen is expressed on virtually all B-lineage malignancies from lymphoblastic leukemia (ALL) to non-Hodgkin's lymphoma (NHL).

Antigen-binding molecules according to the invention wherein the tumor specificity is towards CD30 may be particularly useful in treating Hodgkin's disease and T-cell lymphomas.

For increasing serum-half life of the antigen-binding molecule according to the invention in the body, the antigen-binding molecule, if desired, may be fused to albumin, e.g. HSA, or pegylated, sialylated or glycosylated (see, for example, Stork et al., 2008, J. Biol. Chem., 283:7804-7812).

In some embodiments the trispecific antigen-binding molecule comprises at least one antigen binding site, wherein the VH and VL domains of the VH/VL pair of the antigen binding site are non-covalently bonded with one another, i.e. the VH and VL domains of this VH/VL pair are not linked by a peptide linker or a peptide bond. These non-covalently bonded VH and VL domains are located on different, i.e. a first and a second, polypeptide chains of a multimeric antigen-binding molecule. In some embodiments each of these non-covalently bonded VH and VL domains of this antigen binding site is bonded by a peptide linker or peptide bond to a VH or a VL domain of a second VH/VL pair of a juxtaposed antigen binding site. Preferably, such peptide linker to a VH or a VL domain of a VH/VL pair of a juxtaposed antigen binding site is short for preventing intramolecular folding between the juxtaposed domains and for forcing the association of the two non-covalently bonded VH and VL domains with each other. For example the peptide linker comprises 12 or less amino acid residues, preferably 3 to 9 amino acid residues. Such a generation of at least one antigen binding site by two non-covalently bonded VH and VL domains is advantageous for the stability of the antigen-binding molecule, because it leads to a more compact antigen-binding molecule.

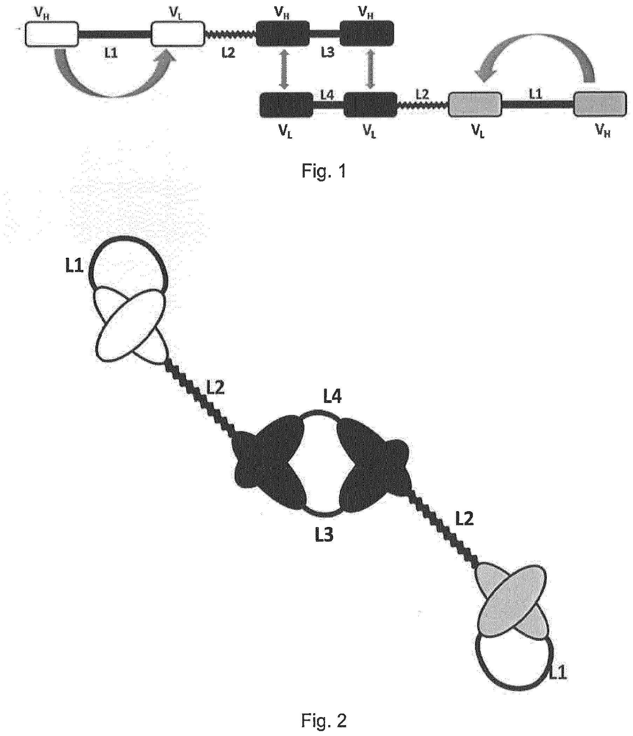

For example FIGs. 1 and 2 show trispecific antigen-binding molecule wherein the VH and VL domains of the central VH/VL pairs (illustrated in black) are non-covalently bond with one another. In this example the non-covalently bonded VH and VL domains are located on different polypeptide chains. Each of these non-covalently bonded VH and VL domains of this antigen is bonded by a peptide linker L3 or L4 to a VH or a VL domain of a second VH/VL pair of a juxtaposed antigen binding site.

In further embodiments the trispecific antigen-binding molecule comprises at least one first antigen binding site, wherein the VH and VL domains of the VH/VL pair of this first antigen binding site are non-covalently bonded with one another, i.e. the VH and VL domains of this VH/VL pair are not linked by a peptide linker or a peptide bond and the non-covalently bonded VH domain of this first antigen binding site is bonded by a peptide linker to a VH domain of a VH/VL pair of a second antigen binding site located juxtaposed to the first antigen-binding site and the non-covalently bonded VL domain of the first antigen binding site is bonded by a peptide linker to a VL domain of a VH/VL pair of the second antigen binding site located juxtaposed to the first antigen-binding site. In embodiments where the antigen-binding molecule is a multimeric, i.e. multi-chain, polypeptide, the VH domain of the first antigen binding site bonded by a peptide linker or peptide bond to a VH domain of the second antigen site are located on a first polypeptide and the VL domain of the first antigen binding site bonded by a peptide linker or peptide bond to a VL domain of a second antigen binding site are located on a second polypeptide. Preferably, the peptide linker is short, e.g. less than 12 amino acid residues, preferable 3 to 9 amino acid residues, for preventing intramolecular folding between the juxtaposed VH-VH and juxtaposed VL-VL domains, respectively, and forcing the association of the VH-VH domains with the VL-VL domains for forming the first and the second antigen binding sites. This VH-VH and VL-VL domain arrangement facilitates the correct folding of the trispecific antigen-binding molecule.

"Antigen-binding molecule" refers to a molecule of an immunoglobulin derivative with multivalent antigen-binding properties, preferably having at least four antigen-binding sites. The antigen-binding molecule is a multimeric polypeptide. Each polypeptide of the antigen-binding molecule comprises antibody variable (Fv) domains linked with one another by a peptide linker or a peptide bond. Each antigen-binding site is formed by an antibody, i.e. immunoglobulin, variable heavy domain (VH) and an antibody variable light domain (VL) binding to the same antigen epitope. The antigen epitope may be on the same or different antigens. Preferably, the antigen-binding molecule according to the invention is devoid of immunoglobulin constant domains or fragments thereof.

The term "polypeptide" refers to a polymer of amino acid residues linked by amide bonds. The polypeptide is, preferably, a single chain fusion protein which is not branched. Within the polypeptide the antibody variable (Fv) domains are linked one after another. The polypeptide may have contiguous amino acid residues in addition N-terminal and/or C-terminal. For example, the polypeptide may contain a Tag sequence, preferably at the C-terminus which might be useful for the purification of the polypeptide. Example of a Tag sequence are a His-Tag, e.g. a His-Tag consisting of six His-residues, a FLAG, e.g. a DYKDDDDK octapeptide (SEQ ID NO:5) or STREP^{®} II, e.g a WSHPQFEK octapeptide (SEQ ID NO:6). For a multimeric antigen-binding molecule, preferably, different Tag sequences are used for different polypeptides.

Regarding the amino acid composition of the peptide linkers, peptides are selected that do not interfere with the association of the domains as well as do not interfere with the multimerization, e.g. dimerization, of multimeric molecules. For example, linkers comprising glycine and serine residues generally provide protease resistance. The amino acid sequence of the linkers can be optimized, for example, by phage-display methods to improve the antigen binding and production yield of the antigen-binding molecule. In an embodiment (G₂S)ₓ peptide linkers are used.

In some embodiments of the invention at least one, preferably all, antibody variable domains are fully human, humanized or chimeric domains. Humanized antibodies can be produced by well-established methods such as, for example CDR-grafting (see, for example, Antibody engineering: methods and protocols / edited by Benny K.C. Lo; Benny K.C. II Series: Methods in molecular biology (Totowa, N.J.). Thus, a skilled person is readily able to make a humanized or fully human version of antigen-binding molecule and variable domains from non-human, e.g. murine or non-primate, sources with the standard molecular biological techniques known in the art for reducing the immunogenicity and improving the efficiency of the antigen-binding molecule in a human immune system. In a preferred embodiment of the invention all antibody variable domains are humanized or fully human; most preferred, the antigen-binding molecule according to the invention is humanized or fully human. The term "Fully human" as used herein means that the amino acid sequences of the variable domains and the peptides linking the variable domains in the polypeptide originate or can be found in humans. In certain embodiments of the invention the variable domains may be human or humanized but not the peptides linking the antibody variable domains.

The present invention provides a multifunctional antigen-binding polypeptide multimer.

The present invention provides an antigen-binding molecule of a trifunctional antigen-binding polypeptide multimer designed to target three different antigens or epitopes. Such a multimer comprises a first polypeptide and a second polypeptide. Each of the two polypeptides is a single-chain fusion peptide having at least four antibody variable domains linked one after another from the N- to the C-terminus of each polypeptide. Each of the polypeptides comprises two antibody variable domains linked by a short linker for preventing intramolecular pairing within the same polypeptide and a single-chain Fv unit having an antibody variable domain pair of the other two variable domains capable of intramolecularly forming an antigen binding site by the variable domain pair within the same polypeptide. The multimer is formed by non-covalent association between the two polypeptides, whereas the two antibody variable domains linked by a short linker of one polypeptide associate with the two corresponding antibody variable domains of the other polypeptide, thereby forming two additional antigen binding sites. Thus, this multimer comprises at least four antigen binding sites and is at least tetravalent. The multimer is a dimer, i.e. consists of two polypeptide chains.

For generating such a trispecific and tetravalent antigen-binding dimer the two polypeptides have to be of different antibody variable domain compositions, because with respect to at least one of the three specificities the respective antibody variable light domain and variable heavy domain have to be inserted into different polypeptides such that one of the polypeptides contains only the variable heavy domain and the other polypeptide contains only the variable light domain for this specificity. Thus, such a dimer according to the invention is heterodimeric, because it is composed of two different polypeptides.

Particular measures can be taken for enabling a correct association of the two different polypeptides comprising antibody variable domains for three different specificities and to prevent a wrong homodimerization between two identical polypeptides. For example, the inventors have obtained a correct heterodimerization between the two different, trispecific polypeptides by inserting two antibody variable heavy domains linked by a short linker in one polypeptide and inserting the two corresponding antibody variable light domains linked by a short linker into the other polypeptide. Surprisingly, only heterodimeric species of the trispecific antigen-binding polypeptide dimers have been formed.

Therefore, the invention provides a trispecific antigen-binding molecule, wherein said molecule is a trispecific antigen-binding polypeptide dimer comprising a first polypeptide and a second polypeptide, each polypeptide having at least four antibody variable domains linked one after another, and
(a) the first polypeptide comprises a first and a second antibody variable heavy domain (VH) linked with each other by a first linker preventing intramolecular pairing within the same polypeptide, for example of about 12 or less amino acid residues, and a single chain Fv antigen binding unit having a third antibody variable heavy domain (VH) linked by a second peptide linker with an antibody variable light domain (VL), said third antibody variable heavy domain (VH) and antibody variable light domain (VL) are capable to associate to a first antigen binding site, wherein the second antibody variable heavy domain (VH) is linked with the single chain Fv antigen binding unit by a third peptide linker; and
(b) the second polypeptide comprises a first and a second antibody variable light domain (VL) linked with each other by a second peptide linker preventing intramolecular pairing within the same polypeptide, for example of about 12 or less amino acid residues, and a single chain Fv antigen binding unit having a third antibody variable domain (VL) linked by a second peptide linker with an antibody variable heavy domain (VH), said third antibody variable light domain (VL) and antibody variable heavy domain (VH) are capable to associate to a second antigen binding site, wherein the second antibody variable light domain (VL) is linked with the single chain Fv antigen binding unit by a third peptide linker; and
(c) the first and the second antibody variable heavy domain (VH) of the first polypeptide associate with the first and the second antibody variable light domain (VL) of the second polymer to two additional, i.e. a third and forth, antigen binding sites, whereas in a preferred embodiment the first antibody variable heavy domain (VH) of the first polypeptide associates with the second antibody light chain region (VL) of the second polypeptide to a third antigen binding site and the second antibody variable heavy domain (VH) of the first polypeptide associates with the first antibody variable light domain (VL) of the second polypeptide to a fourth antigen binding site.

A trispecific antigen-binding polypeptide dimer is formed, when two of said four antigen binding sites are specific for the same antigen.

Such a trispecific dimer recognizes three different specificities, and can target, for example, two different antigens or epitopes on a target cell and with the third functionality, i.e. specificity, bind, for example, to an immune effector cell such as, for example, a T- or a NK-cell.

The trispecific, dimer according to the invention can be utilized in different ways.

For example, the antibody variable domains may be arranged within a polypeptide such that the two antibody variable domains associating with the two corresponding antibody variable domains of the other polypeptide may be positioned, for example, at the N-terminus or the C-terminus of the polypeptide. These two antibody variable domains may have the same specificity or distinct specificities. For example, both may be specific for the same immune effector cell or have distinct specificities for two antigens on a tumor cell.

Further, the two antibody variable domains forming the single-chain Fv unit may be, for example, in the order VH-VL or VL-VH in the direction from the N- to the C-terminus of the polypeptide. The single-chain Fv units of the two dimerized polypeptides may have the same or different specificities. For example, if the two antibody variable domains associating with the two corresponding antibody variable domains of the other polypeptide have the same specificity, the single-chain Fv units of the two polypeptides have different specificities for achieving a trispecific dimer.

Thus, the at least four antibody variable domains may be arranged, for example, such that the two antibody variable domains associating with the two corresponding antibody variable domains of the other polypeptide are specific for an immune effector cell and the single-chain Fv units of the two polypeptides have specificities for two distinct tumor antigens or the two antibody variable domains associating with the two corresponding antibody variable domains of the other polypeptide are specific for distinct tumor antigens and both single-chain Fv units of the two polypeptides have the same specificity for an immune effector cell.

The antigen-binding polypeptide is a "dimer" which term refers to a complex of a first and a second polypeptide monomer. In one aspect the antigen-binding polypeptide dimer is a "heterodimer" which term means that the antigen-binding polypeptide is composed of two different polypeptide monomers that are encoded by two distinct polynucleotides.

Preferably, in the antigen-binding dimer the first and the second polypeptides are non-covalently associated with each other, in particular with the proviso that there is no covalent bound between the first and second polypeptide. However, if desired, the two polypeptides may be additionally stabilized by at least one covalent linkage, e.g. by a disulfide bridge between cysteine residues of different polypeptides.

The length of the linkers influences the flexibility of the antigen-binding polypeptide dimer. The desired flexibility of the antigen-binding polypeptide dimer depends on the target antigen density and the acessibility of the target antigen, i.e. epitopes. Longer linkers provide a more flexible antigen-binding polypeptide dimer with more agile antigen-binding sites. The effect of linker length on the formation of dimeric antigen-binding polypeptides is described, for example, in Todorovska et al., 2001 Journal of Immunological Methods 248:47-66; Perisic et al., 1994 Structure 2:1217-1226; Le Gall et al., 2004, Protein Engineering 17:357-366 and WO 94/13804.

According to the invention it is preferred that the length of the first peptide linker of the first and second antibody variable heavy domains of the first polypeptide and the first and second antibody variable light domains of the second polypeptide is such that the domains of the first polypeptide can associate intermolecularly with the domains of the second polypeptide to form the dimeric antigen-binding polypeptide. Such linkers are "short", i.e. consist of 0, 1, 2, 3, 4, 5, 6, 7, 8, 9, 10, 11 or about 12 amino acid residues. In the case of 0 amino acid residues the linker is a peptide bond. Such short linkers favor the correct dimerization of the first with the second polypeptide by binding and forming antigen-binding sites between antibody variable light domains and antibody variable heavy domains of different polypeptides. Shortening the linker to about 12 or less amino acid residues generally prevents adjacent domains of the same polypeptide chain from interacting with each other. In an embodiment of the invention these linkers consist of about 3 to about 10, for example 7 contiguous amino acid residues. Besides, it is in principle possible that two polypeptides having a linker with more than 12 amino acid residues between the variable antibody domains correctly dimerize with one another (see for example Le Gall et al., 2004, Protein Engineering 17:357-366).

For the single-chain Fv units of the polypeptides the second peptide linker is long and flexible (in general consisting of about 12 or more amino acid residues) for folding intramolecularly head-to-tail and forming the single-chain antigen-binding (scFv) unit. Additional amino acid residues provide extra flexibility. For example this linker between the VH and VL or VL and VH of the single-chain Fv unit in the polypeptide may consist of about 12 to about 35, in particular from 15 to 25 contiguous amino acid residues.

The third peptide linker of the polypeptide for linking the single-chain Fv unit with the other two antibody variable domains which associate with the corresponding variable domains of the other polypeptide may be, for example, from 5 to 30, preferably at least 6, 7, 8, 9, 10, 11, or 12 contiguous amino acid residues.

In an embodiment of the invention the trispecific antigen-binding polypeptide dimer is bispecific for two distinct antigens on a tumor cell and additionally specific for an effector cell, in particular a T cell or a NK cell. Suitable specificities for tumor cells may be tumor antigens and cell surface antigens on the respective tumor cell, for example specific tumor markers. Such a trispecific antigen-binding dimer binds bifunctionally to a tumor cell and to the immune effector cell thereby triggering the cytotoxic response induced by the T cell or the NK cell.

The antigen-binding molecule according to any one of the embodiments described here previously may be produced by expressing polynucleotides encoding the individual polypeptide chains which form the antigen-binding molecule. Therefore, a further embodiment of the invention are polynucleotides, e.g. DNA or RNA, encoding the polypeptide chains of the antigen-binding molecule as described herein above.

The polynucleotides may be constructed by methods known to the skilled person, e.g. by combining the genes encoding the antibody variable domains either separated by peptide linkers or directly linked by a peptide bound of the polypeptides, into a genetic construct operably linked to a suitable promoter, and optionally a suitable transcription terminator, and expressing it in bacteria or other appropriate expression system such as, for example CHO cells. Depending on the vector system and host utilized, any number of suitable transcription and translation elements, including constitutive and inducible promoters, may be used. The promoter is selected such that it drives the expression of the polynucleotides in the respective host cell.

The polynucleotides may be inserted into vectors, preferably expression vectors, which represent a further embodiment of the invention. These recombinant vectors can be constructed according to methods well known to the person skilled in the art. A variety of expression vector/host systems may be utilized to contain and express the polynucleotides encoding the polypeptide chains of the present invention. Examples for expression vectors for expression in *E.coli* is pSKK (LeGall et al., J Immunol Methods. (2004) 285(1):111-27) or pcDNA5 (Invitrogen) for the expression in mammal cells.

Thus, the antigen-binding molecule as described herein may be produced by introducing a vector encoding the polypeptide chains as described above into a host cell and culturing said host cell under conditions whereby the polypeptide chains are expressed, may be isolated and, optionally, further purified.

In a further embodiment of the invention compositions comprising a antigen-binding moleculeor polynucleotides as described herein above and at least one further component are provided.

The invention further provides a method wherein the antigen-binding molecule as described herein above is administered in an effective dose to a subject, e.g., patient, for the treatment of cancer (e.g. non-Hodgkin's lymphoma; chronic lymphocytic leukaemia). The antigen-binding molecule can be used as a medicament.

A skilled person will readily be able without undue burden to construct and obtain the antigen-binding molecule described herein by utilizing established techniques and standard methods known in the art, see for example Sambrook, Molecular Cloning A Laboratory Manual, Cold Spring Harbor Laboratory (1989) N.Y.; The Protein Protocols Handbook, edited by John M. Walker, Humana Press Inc. (2002); or Antibody engineering: methods and protocols / edited by Benny K.C. Lo; Benny K.C. II Series: Methods in molecular biology (Totowa, N.J.)).

### Brief description of the figures:

Figure 1 shows a first and a second polypeptide for forming a trifunctional, i.e. trispecific, antigen-binding polypeptide dimer according to the invention. The first polypeptide has four antibody variable domains VH, VL, VH, VH linked one after another. The first and the second VH antibody variable domains (black) have the same first specificity and are linked by a short linker L3 for preventing intramolecular pairing within the same polypeptide and a single-chain Fv unit having an antibody variable domain pair of the other third variable antibody domain VL and the fourth variable antibody domain VH (white) linked by a second linker L1 capable of intramolecularly forming an antigen binding site of a second specificity by the variable domain pair within the same polypeptide. The second antibody variable domain VH and the third antibody variable domain VL of different specificities are linked by a third linker L2.
   The second polypeptide has four antibody variable domains VL, VL, VL, VH linked one after another The first and the second VL antibody variable domains (black) have the same first specificity and are linked by a short linker L4 for preventing intramolecular pairing within the same polypeptide and a single-chain Fv unit having an antibody variable domain pair of the other third variable antibody domain VL and the fourth variable antibody domain VH having a third specificity (grey) and are linked by a second linker L1 capable of intramolecularly forming an antigen binding site by the variable domain pair within the same polypeptide. The second antibody variable domain VL and the third antibody variable domain VL of different specificities are linked by a third linker L2.
Figure 2 shows the antigen-binding polypeptide dimer formed by non-covalent association between the two polypeptides of Figure 1, whereas the two antibody variable VH domains linked by a short linker of the first polypeptide associate with the two corresponding antibody variable VL domains of the second polypeptide, thereby forming two antigen binding sites having the same specificity (black), whereas the second specificity is provided by the single chain Fv unit of the first polypeptide (white) and the third specificity is provided by the single chain Fv unit of the second polypeptide (grey).

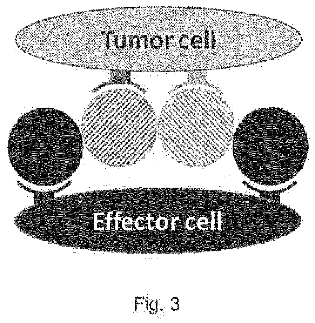

Figure 3 shows a trifunctional antigen-binding molecule, in particular trifunctional antigen-binding polypeptide, according to the invention which is a trispecific antibody for dual targeting of tumor cells. The antibody, i.e. antigen-binding polypeptide, is designed to target two different targets/epitopes on the tumor cell and with the third functionality bind with high affinity to an effector cell. The antigen-binding polypeptide consists of four antigen binding sites, wherein the two central antigen binding sites bind to two different antigens on the tumor cell and the two peripheral antigen binding sites bind to the effector cell.

The examples below further illustrate the invention without limiting the scope of the invention.

### Example 1

### DNA constructs:

The plasmid DNA encoding the polypeptide chains are generated by DNA engineering or by gene synthesis and sequencing. The expression constructs for transient or stable transfection of mammalian cells are based on the eukaryotic expression vector pCDNA5/FRT (Life Technologies) and comprise the product gene of interest under the control of a viral or ubiquitous promoter, as well as a Hygromycin resistance cassette as a selection marker. For purification and analytics, the product chains are expressed with His-tag, FLAG-tag or Strepll-tag.

### Cell Lines and Cell Cultivation:

Flp-In CHO cells (Life Technologies), a derivative of CHO-K1 Chinese Hamster ovary cells (ATCC, CCL-61) (Kao and Puck, 1968), are cultured in Ham's F-12 Nutrient Mix supplemented with L-Glutamine, 10% FCS and 100 µg/ml Zeocin. Adherent cells are detached with 0.25% Trypsin-EDTA and subcultured according to standard cell culture protocols.

For adaptation to growth in suspension, cells are detached from tissue culture flasks and placed in serum-free medium for subsequent incubation in shake flasks (Corning) at 37°C, 5% CO₂ and 120 rpm. The standard medium for the culture of suspension-adapted Flp-In CHO cells is HyClone CDM4 CHO (Thermo Scientific) supplemented with L-Glutamine (Life Technologies), HT Supplement (Life Technologies), Penicillin/Streptomycin (Life Technologies) and 100 µg/ml Zeocin (Life Technologies). Suspension-adapted cells are subcultivated every 2-3 days with seeding densities of 2E+6 to 3E+6 cells/ml. The cell concentration and viability is determined in all cultures using the trypan blue exclusion method. Cells are cryopreserved in medium with 10% DMSO and tested negative for Mycoplasma using MycoAlert Mycoplasma detection Kit (Lonza).

### Generation of stably transfected cell pools:

Recombinant Flp-In CHO cell lines stably expressing tri-specific candidate antibodies, are generated by transfection of suspension-adapted cells. For this, cells are placed in standard medium without Zeocin one day prior to co-transfection with expression plasmids (2.5 µg) encoding the protein of interest (pcDNA5/FRT) and the Flp recombinase (pOG44, Life Technologies) using Polyethylenimine (PEI). In brief, vector DNA and transfection reagent are mixed at a DNA:PEI mass ratio of 1:3 in a total of 100 µL OptiMEM I medium (Life Technologies) and incubated for 10 minutes before addition to 2E+6 Flp-In CHO cells suspended in 1ml CHO-S-SFMII medium (Life Technologies). Following 24h incubation, selection for stably transfected cells is started by addition of 500µg/mL Hygromycin B subsequent to diluting cultures to a density of 0.1E+6 viable cells/mL in CHO-S-SFMII medium and seeding in T75 culture flasks. Flp recombinase mediates the insertion of the Flp-In expression construct into the genome at the integrated FRT site through site-specific DNA recombination (O' Gorman et al 1991). During selection viable cell densities are measured twice a week, and cells are centrifuged and resuspended in fresh selection medium at a maximal density of 0.1 E+6 viable cells/mL.Cell pools stably expressing recombinant protein products are recovered after approximately 3 weeks of selection at which point cells are transferred to standard culture medium in shake flasks. Expression of recombinant secreted proteins is confirmed by protein gel electrophoresis of cell culture supernatants using Criterion Stain-Free (Bio-Rad) technology. Stable cell pools are cryopreserved in medium containing 50% ProFreeze (Lonza) and 7.5% DMSO.

### Production of recombinant protein in Fed-batch CHO cell suspension cultures :

Recombinant proteins are produced in 10-day fed-batch cultures of stably transfected CHO cell lines by secretion into the cell culture supernatant. For this, cell pools stably expressing the product of interest are seeded at starting densities of 6E+5 cells/mL in standard culture medium in polycarbonate Erlenmeyer flasks with gas permeable caps (Corning) and incubated at 37°C and 5% CO2 with agitation at 140 rpm. During fed-batch culture, media is supplemented with 40 mL/L ActiCHO Feed A (PAA) and 4 mL/L ActiCHO Feed B (PAA) on day 0 (starting day), and with double amounts on day 3, 5, and 7. Cell culture supernatants are harvested after 10 days at culture viabilities of typically >75%. Samples are collected from the production cultures every other day prior to feeding and cell density and viability is assessed. On the day of harvest, cell culture supernatants are cleared by centrifugation and vacuum filtration (0.22 µm) using Millipore Express PLUS Membrane Filters (Millipore) before further use.

### Determination of expression titer:

Protein expression titers and product integrity in cell culture supernatants (CCS) are analysed by SDS-PAGE using the Criterion Stain-Free gel imaging system (Bio-Rad) on days 7 and 10 (before and after 0.22 µm filtration). Product titers are determined semi-quantitatively by comparison with a reference protein of known concentration.

### Purification of trispecific antigen-binding polypeptides :

His-tagged products are purified from CHO cell culture supernatants in a two-step procedure comprising Ni-NTA- and preparative size-exclusion chromatography. First, supernatants are cleared by vacuum filtration (0.22 µm) and adjusted to 5 mM imidazole before loading onto HisTrap FF chromatography column (GE Healthcare) equilibrated in IMAC Buffer A at a flow rate of 5 mL/min. Columns are subsequently washed with 5 CV IMAC Buffer A and 10 CV of a mixture of IMAC Buffer A and IMAC Buffer B (7%). His-tagged products are then eluted by sequential washing with 10 CV 30% IMAC Buffer B and 5 CV 100% IMAC Buffer B at the same flow rate. 2.5 mL eluate fractions are collected and protein content and purity is assessed by subjecting each fraction to one-dimensional SDS-PAGE followed by visualization of protein using Criterion Stain-Free technology (Bio-Rad). Product containing fractions are pooled and concentrated by ultrafiltration. Subsequently, concentrated samples are purified by gel filtration using a HiLoad 26/600 Superdex 200 pg (GE Healthcare) column and eluted in SEC Buffer (20 mM Tris-HCl, 100 mM NaCl, pH 7.5) at 2.5 mL/min. Fractions containing the purified product, as determined by comparison of elution volumes with column retention of molecular weight marker proteins (GE Healthcare), are collected and pooled. After a final buffer exchange (10 mM sodium acetate, pH 5.0) using PD-10 desalting columns (GE Healthcare) samples are concentrated to 1.0 - 1.5 mg/mL by ultrafiltration as described above. Purity and homogeneity (typically >90%) of final samples are assessed by Criterion Stain-Free gel visualization of proteins after reducing and non-reducing SDS-PAGE as described above, in selected cases followed by immunoblotting with specific antibodies and by analytical SEC, respectively. Purified proteins are stored as aliquots at -80°C until further use.

### Examples 2 CD3xCD19xCD30 trispecific molecules

Antigen-binding polypeptide dimers containing CD3-, CD19- and CD30-antibody variable binding domains originating from the antibodies OKT3, HD37 and HRS3, respectively are produced according to Example 1:
Trispec 1:
   VH(CD3)-(G₂S)₂- VH(CD3) -(G₂S)₃-VH(CD30)- (G₂S)₅-VL(CD30)-His₆ (SEQ ID NO:1)
   VL(CD3)- (G₂S)₂-VL(CD3)- (G₂S)₃-VH(CD19)- (G₂S)₅VL(CD19)-FLAG (SEQ ID NO:2)
Trispec 2:
   VH(CD30)-(G₂S)₂-VH(CD19)-(G₂S)₂-VH(CD3)-(G₂S)₅-VL(CD3)-His₆ (SEQ ID NO:3)
   VL(CD19)-(G₂S)₂-VL(CD30)- (G₂S)₂-VH(CD3)-(G₂S)₅-VL(CD3)-FLAG (SEQ ID NO:4)
Linker 1 = (G₂S)₂, Linker 2 = (G₂S)₅, Linker 3 = (G₂S)₃

Immunoprecipitation of Trispec 1 and Trispec 2 show that only heterodimeric species of the antigen-binding polypeptide dimer are detected. Trispec 1 and Trispec 2 exhibit excellent stability at 40°C after 7 days and at pH 3.5 after 1h.

### Example: Assessment of cytotoxic activity mediated by trispecific antibodies

### Study procedures

### Isolation of PBMC from buffy coats and enrichment of T cells:

PBMCs are isolated from buffy coats by density gradient centrifugation. T cells are enriched from the PBMC population using the EasySep^{™} Human T Cell Enrichment Kit for the immunomagnetic isolation of untouched human T cells and the Big Easy EasySep^{™} Magnet according to the manufacturer's instructions.

### FACS-based cytotoxicity assay:

T cells that are used as effector cells are characterized by flow cytometry as described.

Target cells (MEC-1: DSMZ, cat.: ACC 497; NALM-6: DSMZ, cat.: ACC 128) are cultured under standard conditions as described below. For the cytotoxicity assay target cells are harvested, washed twice with RPMI 1640 medium without FCS, and resuspended in diluent C provided in the PKH67 Green Fluorescent Cell Linker Mini Kit to a density of 2x10⁷/mL. The cell suspension is then mixed with the equal volume of a double-concentrated PKH67-labeling solution (e.g. 1 µL PKH67 in 250 µL diluent C) and incubated according to the manufacturer's instructions. The staining reaction is stopped. After washing the labeled target cells with complete RPMI medium, cells are counted and resuspended to a density of 2x10⁵/mL in complete RPMI medium.

2x10⁴ target cells are then seeded together with T cells at and the indicated antibodies in individual wells. Spontaneous cell death and killing of targets by effectors in the absence of antibodies are determined.

After incubation, cultures are washed once with FACS buffer and then resuspended in 150 µL FACS buffer supplemented with 2 µg/mL PI. The absolute amount of living target cells that are characterized by a positive green PKH67 staining but are negative for the PI staining are measured using a Beckman-Coulter FC500 MPL flow cytometer (Beckman-Coulter) or a Millipore Guava EasyCyte flow cytometer (Merck Millipore).

Based on the measured remaining living target cells, the percentage of specific cell lysis is calculated according to the following formula: [1-(number of living targets (sample)) / (number of living targets ₍ₛₚₒₙₜₐₙₑₒᵤₛ₎)] x 100%. Sigmoidal dose response curves and EC₅₀ values are calculated by non-linear regression/4-parameter logistic fit using the GraphPad Prism software (GraphPad Prism version 6.00 for Windows, GraphPad Software, La Jolla California USA).

### Statistical analysis

The lysis values obtained for a given antibody concentration are determined and analysed by sigmoidal dose-response/4 parameter logistic fit analysis using the Prism software (GraphPad Prism version 6.00 for Windows, GraphPad Software, La Jolla California USA) and used to calculate EC₅₀ values, and mean and SD of replicates of percentage lysis.

### Results:

Trispec 1 and Trispec 2 exhibit higher cytotoxic potency on double-positive cell lines (CD19⁺ and CD30⁺) when compared to the respective single-positive cell lines.

## Claims

1. A trispecific antigen-binding molecule, wherein said molecule is an antigen-binding polypeptide dimer comprising a first polypeptide and a second polypeptide, each polypeptide having at least four antibody variable domains linked one after another, wherein
(a) the first polypeptide comprises a first and a second antibody variable heavy domain (VH) linked with each other by a linker of 12 or less amino acid residues and a single chain Fv antigen binding unit having a third antibody variable heavy domain (VH) linked with an antibody variable light domain (VL), said third antibody variable heavy domain (VH) and antibody variable light domain (VL) are capable to associate to a first antigen binding site, wherein the second antibody variable heavy domain (VH) is linked with the single chain Fv antigen binding unit by a peptide linker;
(b) the second polypeptide comprises a first and a second antibody variable light domain (VL) linked with each other by a linker of 12 or less amino acid residues and a single chain Fv antigen binding unit having a third antibody variable light domain (VL) linked with an antibody variable heavy domain (VH), said third antibody variable light domain (VL) and antibody variable heavy domain (VH) are capable to associate to a second antigen binding site, wherein the second antibody variable light domain (VL) is linked with the single chain Fv antigen binding unit by a peptide linker;
(c) the first antibody variable heavy domain (VH) of the first polypeptide associates with the second antibody variable light domain (VL) of the second polypeptide to a third antigen binding site;
(d) the second antibody variable heavy domain (VH) of the first polypeptide associates with the first antibody variable light domain (VL) of the second polypeptide to a fourth antigen binding site; and
(e) two of said four antigen binding sites are specific for the same antigen.

2. The antigen-binding molecule according to claim 1, wherein the antigen-binding molecule has specificity to a target selected from the group consisting of immune effector cells, tumor cells, viral or bacterial pathogens.

3. The antigen-binding molecule according to claim 1 or 2, wherein the first and second antigen binding sites of the two single chain Fv units are specific for the same antigen.

4. The antigen-binding molecule according to claim 1 or 2, wherein the third and the fourth antigen binding sites formed by association between the first and the second polypeptide are specific for the same antigen.

5. The antigen-binding molecule according to anyone of claims 1 to 4, wherein the two antigen binding sites specific for the same antigen are specific for an antigen presented on a T-cell or natural killing (NK) cell.

6. The antigen-binding molecule according to anyone of claims 2 to 5, wherein the specificity to an immune effector cell is selected from the group consisting of CD3, CD16 or CD16A.

7. The antigen-binding molecule according to claim 5 or 6, wherein the other two binding sites are specific for two different antigens on the same cell.

8. The antigen-binding molecule according to claim 7, wherein the cell is a tumor cell.

9. The antigen-binding molecule according to claim 8, wherein the two different antigens are selected from the group consisting of CD19, CD20, CD26, CD29, CD30 CD33, CD200, CD267, EGFR, , EGFRvIII, HER2, HER3, IGFR, IGF-1R, Ep-CAM, PLAP, Thomsen-Friedenreich (TF) antigen, MUC-1 (mucin), CD5, IL4-R alpha, IL13-R, FcεRI and IgE, gpA33, MHCl/peptide complexes.

10. The antigen-binding molecule according to anyone of claims 1 to 4, wherein a first antigen binding site and a second antigen binding site are specific for two different antigen epitopes presented on a natural killing (NK) cell.

11. A vector encoding an antigen-binding molecule according to anyone of claims 1 to 10.

12. A host cell transformed by an vector according to claim 11.

13. An antigen-binding molecule according to claims 8 or 9 for use in tumor therapy.

## Patentansprüche

1. Trispezifisches, Antigen-bindendes Molekül, wobei das Molekül ein Antigen-bindendes Polypeptiddimer ist, das ein erstes Polypeptid und ein zweites Polypeptid umfasst, jedes Polypeptid zumindest vier, nach einander verbundene, variable Antikörperdomänen hat, wobei
(a) das erste Polypeptid umfasst eine erste und eine zweite variable schwere Antikörperdomäne (VH), die miteinander durch einen Linker mit 12 oder weniger Aminosäureresten verbunden sind und eine Einzelketten-Fv-Antikörperbindungseinheit, die eine dritte variable schwere Antikörperdomäne (VH) verbunden mit einer variablen leichten Antikörperdomäne (VL) aufweist, wobei die dritte variable schwere Antikörperdomäne (VH) und die variable leichte Antikörperdomäne (VL) zu einer ersten Antigenbindungsstelle assoziieren können, wobei die zweite variable schwere Antikörperdomäne (VH) mit der Einzelketten-Fv-Antigenbindungseinheit durch einen Peptidlinker verbunden ist;
(b) das zweite Polypeptid umfasst eine erste und eine zweite variable leichte Antikörperdomäne (VL), die miteinander durch einen Linker mit 12 oder weniger Aminosäureresten verbunden sind und eine Einzelketten-Fv-Antikörpereinheit, die eine dritte variable leichte Antikörperdomäne (VL) verbunden mit einer variablen schweren Antikörperdomäne (VH) aufweist, wobei die dritte variable leichte Antikörperdomäne (VL) und die variable schwere Antikörperdomäne (VH) zu einer zweiten Antigenbindungstelle assoziieren können, wobei die zweite variable leichte Antikörperdomäne (VL) mit der Einzelketten-Fv-Antikörperbindungseinheit durch einen Peptidlinker verbunden ist;
(c) die erste variable schwere Antikörperdomäne (VH) des ersten Polypeptids assoziiert mit der zweiten variablen leichten Antikörperdomäne (VL) des zweiten Polypeptids zu einer dritten Antigenbindungsstelle;
(d) die zweite variable schwere Antikörperdomäne (VH) des ersten Polypeptids assoziiert mit der ersten variablen leichten Antikörperdomäne (VL) des zweiten Polypeptids zu einer vierten Antigenbindungsstelle; und
(e) zwei von den vier Antigenbindungsstellen sind spezifisch für das gleiche Antigen.

2. Antigen-bindendes Molekül gemäß Anspruch 1, wobei das Antigen-bindende Molekül spezifisch ist für ein Zielobjekt ausgewählt aus der Gruppe bestehend aus Immuneffektorzellen, Tumorzellen, viralen oder bakterielle Pathogene.

3. Antigen-bindendes Molekül gemäß einem der Ansprüche 1 oder 2, wobei die erste und zweite Antigenbindungsstelle der beiden Einzelketten-Fv-Einheiten spezifisch für das gleiche Antigen sind.

4. Antigen-bindendes Molekül gemäß einem der Ansprüche 1 oder 2, wobei die dritte und die vierte Antigenbindungsstelle gebildet durch Assoziierung zwischen dem ersten und dem zweiten Polypeptid spezifisch für das gleiche Antigen sind.

5. Antigen-bindendes Molekül gemäß einem der Ansprüche 1 bis 4, wobei die beiden Antigenbindungsstellen mit gleicher Antigenspezifität spezifisch für ein Antigen sind, das auf einer T-Zelle oder einer natürlichen Killer-(NK)-Zelle präsentiert wird.

6. Antigen-bindendes Molekül gemäß einem der Ansprüche 2 bis 5, wobei die Spezifität zu einer Immuneffektorzelle ausgewählt ist aus der Gruppe bestehend aus CD3, CD16 und CD16A.

7. Antigen-bindendes Molekül gemäß Anspruch 5 oder 6, wobei die beiden anderen Bindungsstellen spezifisch sind für zwei unterschiedliche Antigene auf der selben Zelle.

8. Antigen-bindendes Molekül gemäß Anspruch 7, wobei die Zelle eine Tumorzelle ist.

9. Antigen-bindendes Molekül gemäß Anspruch 8, wobei die beiden unterschiedlichen Antigene ausgewählt sind aus der Gruppe bestehend aus CD19, CD20, CD26, CD30, CD33, CD200, CD267, EGFR, EGFRvlll, HER2, HER3, IGFR, IGF-1R. Ep-CAM, PLAP, Thomsen-Friedenreich (TF) Antigen, MUC-1 (Mucin), CD5, IL4-R alpha, IL13-R, FcεRI and IgE, gpA33, MHCl/Peptidkomplexe.

10. Antigen-bindendes Molekül gemäß einem der Ansprüche 1 bis 4, wobei eine erste Antigenbindungsstelle und eine zweite Antigenbindungsstelle spezifisch für zwei verschiedene Antigenepitope sind, die auf einer natürlichen Killer-(NK)-Zelle präsentiert werden.

11. Vektor, der ein Antigen-bindendes Molekül gemäß einer der Ansprüche 1 bis 10 kodiert.

12. Wirtszelle, die durch einen Vektor gemäß Anspruch 11 transformiert ist.

13. Antigen-bindendes Molekül gemäß Anspruch 8 oder 9 zur Verwendung in der Tumortherapie.

## Revendications

1. Molécule de liaison à un antigène trispécifique, où ladite molécule est un dimère polypeptidique se liant à un antigène comprenant un premier polypeptide et un second polypeptide, chaque polypeptide possédant au moins quatre domaines variables d'anticorps liés l'un après l'autre, où
(a) le premier polypeptide comprend un premier et un deuxième domaine lourd variable d'anticorps (VH) liés l'un à l'autre par un segment de liaison de 12 résidus d'acides aminés ou moins et une unité de liaison à un antigène de Fv à chaîne unique possédant un troisième domaine lourd variable d'anticorps (VH) lié à un domaine léger variable d'anticorps (VL), lesdits troisième domaine lourd variable d'anticorps (VH) et domaine léger variable d'anticorps (VL) étant capables de s'associer au niveau d'un premier site de liaison à un antigène, où le deuxième domaine lourd variable d'anticorps (VH) est lié à l'unité de liaison à un antigène de Fv à chaîne unique par un segment de liaison peptidique;
(b) le second polypeptide comprend un premier et un deuxième domaine léger variable d'anticorps (VL) liés l'un à l'autre par un segment de liaison de 12 résidus d'acides aminés ou moins et une unité de liaison à un antigène de Fv à chaîne unique possédant un troisième domaine léger variable d'anticorps (VL) lié à un domaine lourd variable d'anticorps (VH), lesdits troisième domaine léger variable d'anticorps (VL) et domaine lourd variable d'anticorps (VH) étant capables de s'associer au niveau d'un deuxième site de liaison à un antigène, où le deuxième domaine léger variable d'anticorps (VL) est lié à l'unité de liaison à un antigène de Fv à chaîne unique par un segment de liaison peptidique ;
(c) le premier domaine lourd variable d'anticorps (VH) du premier polypeptide s'associe au deuxième domaine léger variable d'anticorps (VL) du second polypeptide au niveau d'un troisième site de liaison à un antigène ;
(d) le deuxième domaine lourd variable d'anticorps (VH) du premier polypeptide s'associe au premier domaine léger variable d'anticorps (VL) du second polypeptide au niveau d'un quatrième site de liaison à un antigène ; et
(e) deux desdits quatre sites de liaison à un antigène sont spécifiques pour le même antigène.

2. Molécule de liaison à un antigène selon la revendication 1, où la molécule de liaison à un antigène possède une spécificité pour une cible sélectionnée dans le groupe consistant en des cellules effectrices immunitaires, des cellules tumorales, des agents pathogènes viraux ou bactériens.

3. Molécule de liaison à un antigène selon la revendication 1 ou 2, dans laquelle les premier et deuxième sites de liaison à un antigène des deux unités Fv à chaîne unique sont spécifiques pour le même antigène.

4. Molécule de liaison à un antigène selon la revendication 1 ou 2, dans laquelle le troisième et le quatrième site de liaison à un antigène formés par l'association entre le premier et le second polypeptide sont spécifiques pour le même antigène.

5. Molécule de liaison à un antigène selon l'une quelconque des revendications 1 à 4, dans laquelle les deux sites de liaison à un antigène spécifiques pour le même antigène sont spécifiques pour un antigène présenté sur une cellule T ou une cellule tueuse naturelle (NK).

6. Molécule de liaison à un antigène selon l'une quelconque des revendications 2 à 5, dans laquelle la spécificité pour une cellule effectrice immunitaire est sélectionnée dans le groupe consistant en CD3, CD16 ou CD16A.

7. Molécule de liaison à un antigène selon la revendication 5 ou 6, dans laquelle les deux autres sites de liaison sont spécifiques pour deux antigènes différents sur la même cellule.

8. Molécule de liaison à un antigène selon la revendication 7, où la cellule est une cellule tumorale.

9. Molécule de liaison à un antigène selon la revendication 8, dans laquelle les deux antigènes différents sont sélectionnés dans le groupe consistant en CD19, CD20, CD26, CD29, CD30, CD33, CD200, CD267, l'EGFR, l'EGFRvIII, HER2, HER3, l'IGFR, l'IGF-1 R, Ep-CAM, PLAP, l'antigène de Thomsen-Friedenreich (TF), MUC-1 (mucine), CD5, l'IL4-R alpha, l'IL13-R, FcεRI et une IgE, gpA33, des complexes CMHI/peptide.

10. Molécule de liaison à un antigène selon l'une quelconque des revendications 1 à 4, dans laquelle un premier site de liaison à un antigène et un deuxième site de liaison à un antigène sont spécifiques pour deux épitopes antigéniques différents présentés sur une cellule tueuse naturelle (NK).

11. Vecteur codant pour une molécule de liaison à un antigène selon l'une quelconque des revendications 1 à 10.

12. Cellule hôte transformée par un vecteur selon la revendication 11.

13. Molécule de liaison à un antigène selon les revendications 8 ou 9 destinée à être utilisée dans une thérapie tumorale.
